# EUROPEAN PATENT SPECIFICATION

(11) **EP 0 524 633 B1**
(45) Date of publication and mention of the grant of the patent: **31.03.1999**
(21) Application number: 92112643.9
(22) Date of filing: 24.07.1992
(51) Int. Cl.: A61K 33/00, A61K 33/34

(54) **Pharmaceutical composition suitable for influencing the reticuloendothelial system**
Zur Beeinflussung des retikuloendothelialen Systems geeignetes Arzneimittel
Composition pharmaceutique apte à influencer le système réliculo-endothélial

(30) Priority: 24.07.1991 HU 249291
(43) Date of publication of application: 27.01.1993
(73) Proprietor: BERES EXPORT-IMPORT RT., H-1146 Budapest (HU)
(72) Inventor: Béres, József, Dr., H-4600 Kisvárda (HU); Béres jun., Júzsef, Dr., H-1025 Budapest (HU)
(74) Representative: Beszédes, Stephan G., Dr.

(56) References cited:
- GB-A- 2 022 998

## Description

The present invention is concerned with a pharmaceutical composition or product, respectively, suitable for influencing the reticuloendothelic system and for treating mucoviscidosis and chronic pain syndromes deriving from degenerative locomotor diseases or accompanying diseases of tumorous origin and a process for preparing them.

Hungarian patent specification No. 176,202 and British patent specification No. 2,022,998 describe a pharmaceutical composition suitable for influencing the reticuloendothelic system (RES), i.e. the tissue system built up from different tissue types, situated at different anatomical positions or organs of the human or animal organism. The said composition comprises
i) a mixture of pharmaceutically acceptable, water-soluble compounds of boron, fluorine, magnesium, vanadium, manganese, iron, cobalt, nickel, copper, zinc and molybdenum, which compounds do not precipitate with each other or with the other components of the composition and exhibit a neutral or acidic pH in an aqueous medium,
ii) glycine,
iii) glycerol,
iv) L-(+)-ascorbic acid,
v) a neutral or acidic, water-soluble salt of a 2,4,5,7-tetrahalofluorescein (according to the present nomenclature: 2',4',5',7'-tetrahalofluorescein),
vi) a neutral or acidic and water-soluble, pharmaceutically acceptable salt of ethylenediaminetetraacetic acid
   and
vii) potassium sodium tartrate;
   the weight ratio of the boron compound(s) : fluorine compound(s) : magnesium compound(s) : vanadium compound(s) : manganese compound(s) : iron compound(s) : cobalt compound(s) : nickel compound(s) : copper compound(s) : zinc compound(s) : molybdenum compound(s) : glycine : glycerol : L-(+)-ascorbic acid : 2,4,5,7-tetrahalofluorescein salt : ethylenediaminetetraacetic acid salt : potassium sodium tartrate being 0.01 to 1 : 0.02 to 1 : 0.4 to 3 : 0.02 to 0.6 : 0.1 to 2 : 1 to 6 : 0.1 to 1 : 0.02 to 2 : 0.05 to 1 : 0.1 to 3 : 0.01 to 0.8 : 0.1 to 2 : 2 to 8 : 0.01 to 2 : 0.003 to 0.5 : 0.1 to 3 : 0.7 to 10.

The said composition is prepared by dissolving the components in aqueous medium, mixing them with one or more pharmaceutically acceptable carrier(s), diluent(s) and/or excipient(s) and then transforming the mixture thus obtained into a pharmaceutical composition in a manner known per se.

According to a preferred embodiment boric acid is used as boron compound, sodium fluoride or vanadium trifluoride is used as fluorine compound, magnesium sulfate or magnesium chloride or a hydrate thereof is used as magnesium compound, ammonium vanadate or vanadium trifluoride is used as vanadium compound, manganese sulfate or manganese chloride or a hydrate thereof is used as manganese compound, iron(II)- or iron(III)-sulfate or a hydrate thereof is used as iron compound, cobalt chloride or cobalt sulfate or a hydrate thereof is used as cobalt compound, nickel chloride or nickel sulfate or a hydrate thereof is used as nickel compound, copper(II)-sulfate or a hydrate thereof is used as copper compound, zinc sulfate or a hydrate thereof is used as zinc compound and ammonium molybdenate or sodium molybdenate is used as molybdenum compound.

According to a further preferred embodiment of the process 2',4',5',7'-tetraiodofluorescein disodium salt is used as tetrahalofluorescein salt, the disodium salt of ethylenediaminetetraacetic acid salt is used as ethylenediaminetetraacetic acid salt and distilled water is used to make up the aqueous medium.

The problem underlying to the present invention is to work out a superior pharmaceutical composition or product, respectively, enabling the treatment of mucoviscidosis and chronic pain syndromes deriving from degenerative locomotor diseases or accompanying the diseases of tumorous origine, in addition to exhibiting the advantageous properties of the above mentioned composition and a process for preparing it.

Surprisingly this has been achieved by the present invention.

The invention is based on the surprising recognition that the above can be achieved if the 2',4',5',7'-tetrahalofluorescein salt is omitted from the above-identified composition, while it is supplemented with succinic acid and L-(+)-tartaric acid. The composition thus obtained is excellently suitable for the treatment of the above pain syndromes.

The invention is based on the further surprising recognition that by changing the components of the composition in the above manner, the composition thus obtained potentiates the effect of the conventional pain-killers, such as morphine, used for the treatment of chronic pain syndromes accompanying the tumorous diseases, i.e. the amount and the disadvantageous side-effects of the conventional pain-killers can be reduced, and it favourably influences the tolerance threshold and the degree of addiction.

The invention is based on the further surprising recognition that the advantageous properties of the composition described in the above Hungarian and British patent specifications remain practically unchanged if the lower limit of the weight ratio of certain components is reduced.

Thus, the subject matter of the present invention is a pharmaceutical composition having a pH of 1.9 to 4.0 suitable for influencing the reticuloendothelic system and for treating mucoviscidosis and chronic pain syndromes deriving from degenerative locomotor diseases or accompanying the diseases of tumorous origin, containing
i) one or more pharmaceutically acceptable, water-soluble compounds of boron, fluorine, magnesium, vanadium, manganese, iron, cobalt, nickel, copper, zinc and molybdenum, which compounds do not precipitate with each other or with the other components of the composition and exhibit a neutral or acidic pH in an aqueous medium,
ii) glycine,
iii) glycerol,
iv) L-(+)-ascorbic acid,
v) a neutral or acidic and water-soluble, pharmaceutically acceptable salt of ethylene-diaminetetraacetic acid,
vi) potassium sodium tartrate,
vii) succinic acid
   and
viii) L-(+)-tartaric acid,
   optionally together with one or more usual pharmaceutical carrier(s), diluent(s) and/or auxiliary agent(s).

The pharmaceutical composition according to the invention - similarly to the pharmaceutical composition described in the above-mentioned Hungarian and British patent specifications - is based substantially on water, i.e. the components can be reacted and mixed in aqueous medium.

Preferably the composition according to the invention contains the boron compound(s) [i)] : fluorine compound(s) [i)] : magnesium compound(s) [i)] : vanadium compound(s) [i)] : manganese compound(s) [i)] : iron compound(s) [i)] : cobalt compound(s) [i)] : nickel compound(s) [i)] : copper compound(s) [i)] : zinc compound(s) [i)] : molybdenum compound(s) [i)] : glycine [ii)] : glycerol [iii)] : L-(+)-ascorbic acid [iv)] : ethylenediaminetetraacetic acid salt [vi)] : potassium sodium tartrate [vii)] : succinic acid [vii)] : L-(+)-tartaric acid [viii)] in a weight ratio of 0.01 to 1 : 0.01 to 1 : 0.2 to 3 : 0.01 to 0.6 : 0.02 to 2 : 0.15 to 6 : 0.002 to 1 : 0.01 to 2 : 0.01 to 1 : 0.1 to 3 : 0.001 to 0.8 : 0.1 to 2 : 0.2 to 8 : 0.01 to 2 : 0.01 to 3 : 0.01 to 10 : 0.001 to 2 : 0.1 to 2.

The concentration of components i) - viii) may vary within about 0.001 to 10% by weight/volume.

Furthermore it is preferred that the composition contains boric acid as boron compound [i)].

It is also preferred that it contains sodium fluoride and/or vanadium trifluoride as fluorine compound(s) [i)].

Moreover it is preferred that it contains magnesium sulfate and/or magnesium chloride and/or [a] hydrate(s) thereof, particularly magnesium sulfate heptahydrate, as magnesium compound(s) [i)].

Furthermore it is preferred that it contains ammonium vanadate, particularly ammonium metavandate [NH₄VO₃), and/or vanadium trifluoride as vanadium compound(s) [i)].

It is also preferred that it contains manganese sulfate and/or manganese chloride and/or [a] hydrate(s) thereof, particularly manganese sulfate hydrate and/or manganese chloride tetrahydrate, as manganese compound(s) [i)].

Moreover it is preferred that it contains iron(II)-sulfate and/or iron(III)-sulfate and/or [a] hydrate(s) thereof, particularly iron(II)-sulfate heptahydrate, as iron compound(s) [i)].

Furthermore it is preferred that it contains cobalt chloride and/or cobalt sulfate and/or [a] hydrate(s) thereof, particularly cobalt chloride hexahydrate and/or cobalt sulfate heptahydrate, as cobalt compound(s) [i)].

It is also preferred that is contains nickel chloride and/or nickel sulfate and/or [a] hydrate(s) thereof, particularly nickel sulfate heptahydrate, as nickel compound(s) [i)].

Moreover it is preferred that it contains copper(II)-sulfate or [a] hydrate(s) thereof, particularly copper(II)-sulfate pentahydrate, as copper compound(s) [i)].

Furthermore it is preferred that it contains zinc sulfate and/or [a] hydrate(s) thereof, particularly zinc sulfate heptahydrate, as zinc compound(s) [i)].

It is also preferred that it contains ammonium molybdate [(NH₄)₆Mo₇0₂₄ . 4H₂O/ and/or sodium molybdate [Na₂ Mo0₄ . 2H₂O] as molybdenum compound(s) [i)].

Moreover it is preferred that it contains the disodium salt of ethylenediaminetetraacetic acid and/or [a] hydrate(s) thereof, particularly the dihydrate, as ethylenediaminetetraacetic acid salt [v)].

Preferably the composition according to the invention contains the boron compound(s) [i)] in an amount of 0.01 to 1.0% by weight/volume, calculated on the total volume of the composition.

It is also preferred that it contains the fluorine compound(s) [i)] in an amount of 0.01 to 1.0% by weight/volume, calculated on the total volume of the composition.

Furthermore it is preferred that it contains the magnesium compound(s) [i)] in an amount of 0.2 to 3.0% by weight/volume, calculated on the total volume of the composition.

It is also preferred that it contains the vanadium compound(s) [i)] in an amount of 0.001 to 0.6% by weight/volume, calculated on the total volume of the composition.

Furthermore it is preferred that it contains the manganese compound(s) [i)] in an amount of 0.02 to 2.0% by weight/volume, calculated on the total volume of the composition.

It is also preferred that it contains the iron compound(s) [i)] in an amount of 0.15 to 6.0% by weight/volume, calculated on the total volume of the composition.

Moreover it is preferred that it contains the cobalt compound(s) [i)] in an amount of 0.002 to 1.0% by weight/volume, calculated on the total volume of the composition.

Furthermore it is preferred that it contains the nickel compound(s) [i)] in an amount of 0.01 to 2.0% by weight/volume, calculated on the total volume of the composition.

It is also preferred that it contains the copper compound(s) [i)] in an amount of 0.01 to 1.0% by weight/volume, calculated on the total volume of the composition.

Moreover it is preferred that it contains the zinc compound(s) [i)] in an amount of 0.10 to 3.0% by weight/volume, calculated on the total volume of the composition.

Moreover it is preferred that it contains the molybdenum compound(s) [i)] in an amount of 0.001 to 0.8% by weight/volume, calculated on the total volume of the composition.

The preferable concentration ranges of the other components of the pharmaceutical composition according to the invention are as follows:

| | |
|---|---|
| glycine [ii)] | 0.10 to 2.0% by weight/volume |
| glycerol [iii)] | 0.20 to 8.0% by weight/volume |
| L-(+)-ascorbic acid [iv)] | 0.01 to 2.0% by weight/volume |
| ethylenediaminetetraacetic acid disodium salt [v)] | 0.01 to 3.0% by weight/volume |
| potassium sodium tartrate [vi)] | 0.01 to 10.0% by weight/volume |
| succinic acid [vii)] | 0.01 to 2.0% by weight/volume |
| L-(+)-tartaric acid [viii)] | 0.01 to 2.0% by weight/volume. |

The previous and following data relating to concentrations in % by weight/volume units have to be understood in such a way that the amount of the individual components expressed in grams is dissolved in 100 ml solution.

Preferably the composition of the invention contains as substance(s) for adjusting the pH of the composition to 1.9 to 4.0 one or more pharmaceutically acceptable acid(s) in an amount necessary therefor. Preferably an additional amount of L-ascorbic acid serves to this purpose. As an alternative the composition may contain sulfuric acid and/or hydrochloric acid as pharmaceutically acceptable acid(s). Specifically the pharmaceutically acceptable acid(s) can be present as 1 N acid solutions.

In addition to the above-listed components the pharmaceutical composition according to the invention may comprise one or more dissolution-promoting agent(s), such as ethanol, for example, of 96% by volume, buffer(s) suitable for adjusting the desired, pharmaceutically acceptable pH, for example, consisting of or containing hydrochloric acid and/or sulfuric acid and/or conventional pharmaceutical carrier(s), diluents and/of excipients.

The preferable amount of ethanol used as dissolution-promoting agent is 0.05 to 10.0% by weight/volume, while the preferable amount of the pharmaceutically acceptable acid used for adjusting the pH of the solution is 0.05 to 1.0% by weight/volume.

According to a particular advantageous embodiment of the invention it is provided for a product containing a composition according to the invention and additionally L-(+)-ascorbic acid as a combined preparation for simultaneous, separate or sequential use in the therapy of mucoviscidosis and chronic pain syndromes deriving from locomotor diseases or accompanying the diseases of tumorous origin. Preferably the additional amount of L-(+)-ascorbic acid is from 100 to 300 mg/l to 3 ml of the composition.

According to a further particular advantageous embodiment of the invention it is provided for a product containing a composition according to the invention or containing a product according to the invention and one or more known analgesic and/or narcotic substance(s), particularly morphine, and/or composition(s) as a combined preparation for simultaneous, separate or sequential use in the therapy of chronic pain syndromes deriving from locomotor diseases or accompanying the diseases of tumorous origin. Preferably the amount of the known analgesic and/or narcotic substance(s) and/or composition(s) is less than that usually used, particularly down to the half of it.

A subject-matter of the invention is also a process for preparing the pharmaceutical composition or product according to the invention, which is characterized by reacting the substances i) to viii) in aqueous medium and then optionally mixing the solution thus obtained with an additional amount of L-(+)-ascorbic acid and/or one or more analgesic and/or narcotic substance(s) and/or compositions(s) and/or with one or more pharmaceutically acceptable carrier(s), diluent(s) and/or excipient(s) and forming a pharmaceutical composition.

The pharmaceutical composition according to the invention may be prepared from various complexes which form together a more complicated complex when combined in an aqueous solution while keeping their water-soluble character. The term "more complicated complex" means that, when admixing the complex subgroups, redox reactions and ligand exchange reactions can take place according to the complex-stability constants and the molar ratios of the respective ligands and complex-forming elements (e.g. metal ions). On this basis, after setting of the equilibria, complexes of mixed ligands can be formed which are designated by the term "more complicated complexes" just owing to the complicated relations.

Suitably the composition according to the present invention can be in a form suitable for oral, rectal or vaginal administration. An example of a form for oral administration is the form of drops, an example of a form for rectal admininstration is the form of suppositories and an example of a form for intravaginal administration is the form of pessaries.

When applying the pharmaceutical composition according to the invention in the form of drops for the treatment of mucoviscidosis and chronic pain syndromes deriving from locomotor diseases or accompanying the diseases of tumorous origin a daily dose of 5 to 500 drops, particularly 50 to 150 drops, is preferred.

The aqueous solution obtained by the process according to the invention may be concentrated and the concentrate can be absorbed in a suppository mass to produce suppositories or pessaries. Thus a pharmaceutical composition according to the invention suitable for rectal or vaginal administration is preferably prepared by evaporating the aqueous solution to syrupy consistency and letting it to be absorbed in a suppository mass. The suppositories or pessaries are prepared from this mass in a way conventionally used in the pharmaceutical industry. The suppository mass is preferably prepared from cocoa-butter, carnauba wax or gelatine.

The invention is further illustrated by the following examples.

### Example 1

100 l of pharmaceutical composition is prepared as follows.
1. Preparation of complexing agents (ligands)
   a) 300 g of ethylenediaminetetraacetic acid disodium salt [Selection B₂] are dissolved under heating in 4 l of distilled water.
   b) 200 g of potassium sodium tartrate are dissolved in 2 l of distilled water.
   c) 230 g of glycine are dissolved in 800 ml of distilled water under slight heating, then the solution is supplemented with the copper sulfate and then with the zinc sulfate solutions as described under 4.II.
2. 30 g of L-(+)-ascorbic acid are dissolved in 500 ml of distilled water of a temperature of 50 to 60 °C under shaking.
3. In case of necessity, the pH is adjusted to 1.9 to 4 with L-(+)-ascorbic acid.
4. Preparation of complex groups

### Complex I

Into an Erlenmeyer flask of 1 l volume 200 ml of water, 20 g of sodium fluoride and 20 g of ammonium vanadate are charged. After dissolution the solution is poured into a stirring tank of 100 l volume wherein previously 50 l of distilled water have been charged.

### Complex II

100 g of copper(II)-sulfate pentahydrate are dissolved in 500 ml of distilled water. In an other flask 500 g of zinc sulfate heptahydrate are dissolved in 1 l of distilled water. First the copper sulfate solution, then the zinc sulfate solution are added to the glycine solution prepared according to 1.c). Thus a bluish-violet solution is obtained which is poured under stirring into the complex I being in the tank.

### Complex III

To 1 l of ethylenediaminetetraacetic acid disodium salt [Selection B₂] solution prepared according to 1.a)
- 10 g: of CoCl₂.6H₂0,
- 35 g: of (NH₄)₆Mo₇0₂₄.4H₂0,
- 52 g: of NiSO₄.7H₂0 and
- 8 g: of NH₄V0₃
and 800 ml of distilled water are added and the solid components are dissolved under heating. The solution thus obtained is poured into the solution in the tank.

1000 g of iron(II)-sulfate heptahydrate are separately dissolved in 4 l of distilled water in the presence of 2 l of ethylenediaminetetraacetic acid disodium salt [Selection B₂] solution prepared according to 1.a) and the solution thus obtained is also poured into the tank, then the remaining ethylenediaminetetraacetic acid disodium salt [Selecton B₂] solution is also added under stirring. Thus a bluishgreen, turbid solution is obtained.

### Complex IV

410 g of magnesium sulfate heptahydrate and 50 g of succinic acid are dissolved in 2 l of distilled water under slight heating, then the solution thus obtained is poured under stirring into the solution being in the tank.

### Complex V

94 g of manganese sulfate monohydrate and 50 g of L-(+)-tartaric acid are dissolved in 1 l of distilled water under heating, then the solution thus obtained is poured under stirring into the solution being in the tank.

### Complex VI

60 g of boric acid and 600 g of glycerol (of a concentration of at least 86.4% by weight) are dissolved in the presence of 200 ml of distilled water under slight heating, then the solution thus obtained is poured into the solution being in the tank.
5. Preparation of the composition
   The potassium sodium tartrate solution according to 1.b), then the L-(+)-ascorbic acid solution according to 2 are poured under stirring into the solution being in the tank. The volume of the solution being in the tank is supplemented to 98 l with distilled water. Then the pH of the solution is measured: it must be between 1.9 and 4. If the pH is above 4.0, it is adjusted to about 3.0 by the addition of further amounts of L-ascorbic acid. Finally the volume of the solution is supplemented to 100 litres.
   The solution thus obtained is left to stand for 12 to 24 hours. In the meantime the solution clarifies, its colour turns to yellowish green. After quality control the solution can be formulated.

100 ml of the thus-obtained solution comprises the following components:

| | | |
|---|---|---|
| ammonium metavanadate | NH₄VO₃ | 0.028 g |
| sodium fluoride | NaF | 0.020 g |
| glycine | C₂H₅NO₂ | 0.23 g |
| copper(II)-sulfate | CuSO₄.5H₂O | 0.10 g |
| zinc sulfate | ZnSO₄.7H₂O | 0.50 g |
| ammonium molybdate | (NH₄)₆Mo₇O₂₄.4H₂O | 0.035 g |
| nickel(II)-sulfate | NiSO₄.7H₂O | 0.052 g |
| cobalt(II)-chloride | CoCl₂.6H₂O | 0.010 g |
| ethylenediaminetetraacetic acid salt (EDTA Na₂.2H₂O) | C₁₀H₁₄N₂O₈Na₂.2H₂O | 0.30 g |
| L-(+)-ascorbic acid | C₆H₈O₆ | 0.030 g |
| iron(II)-sulfate | FeSO₄.7H₂O | 1.0 g |
| magnesium sulfate | MgSO₄.7H₂O | 0.41 g |
| succinc acid | C₄H₆O₄ | 0.050 g |
| manganese(II)-sulfate | MnSO₄.H₂O | 0.094 g |
| L-(+)-tartaric acid | C₄H₆O₆ | 0.050 g |
| boric acid | H₃BO₃ | 0.060 g |
| glycerol (87 %) | C₃H₈O₃ | 0.60 g |
| potassium sodium tartrate | C₄H₄O₆KNa.4H₂O | 0.20 g |

### Example 2

The process of Example 1 is followed except that the amount of starting materials is chosen in such a manner that 100 ml of the ready-made solution comprise the following amounts of the components the formulae being as indicated in Example 1:

| | |
|---|---|
| ammonium metavanadate | 0.068 g |
| sodium fluoride | 0.30 g |
| glycine | 1.94 g |
| copper(II)-sulfate | 0.012 g |
| zinc sulfate | 2.85 g |
| ammonium molybdate | 0.020 g |
| nickel(II)-sulfate | 0.070 g |
| cobalt(II)-chloride | 0.0021 g |
| ethylenediaminetetraacetic acid salt (EDTA Na₂.2H₂O) | 0.20 g |
| L-(+)-ascorbic acid | 0.40 g |
| iron(II)-sulfate | 0.153 g |
| magnesium sulfate | 2.94 g |
| succinic acid | 0.030 g |
| manganese(II)-sulfate | 0.23 g |
| L-(+)-tartaric acid | 1.94 g |
| boric acid | 0.30 g |
| glycerol (87 %) | 7.92 g |
| potassium sodium tartrate | 0.10 g |

### Example 3

The process of Example 1 is followed except that the amount of starting materials is chosen in such a manner that 100 ml of the ready-made solution comprise the following amounts of the components the formulae being as indicated in Example 1:

| | |
|---|---|
| ammonium metavanadate | 0.576 g |
| sodium fluoride | 0.95 g |
| glycine | 0.50 g |
| copper(II)-sulfate | 0.99 g |
| zinc sulfate | 1.00 g |
| ammonium molybdate | 0.768 g |
| nickel(II)-sulfate | 1.92 g |
| cobalt(II)-chloride | 0.10 g |
| ethylenediaminetetraacetic acid salt (EDTA Na₂.2H₂O) | 2.97 g |
| L-(+)-ascorbic acid | 1.94 g |
| iron(II)-sulfate | 1.50 g |
| magnesium sulfate | 0.60 g |
| succinic acid | 1.98 g |
| manganese(II)-sulfate | 1.96 g |
| L-(+)-tartaric acid | 0.10 g |
| boric acid | 0.98 g |
| glycerol (87 %) | 1.0 g |
| potassium sodium tartrate | 9.78 g |

### Example 4

The process of Example 1 is followed except that the amount of starting materials is chosen in such a manner that 100 ml of the ready-made solution comprise the following amounts of the components the formulae being as indicated in Example 1:

| | |
|---|---|
| ammonium metavanadate | 0.0011 g |
| sodium fluoride | 0.012 g |
| glycine | 0.11 g |
| copper(II)-sulfate | 0.50 g |
| zinc sulfate | 0.11 g |
| ammonium molybdate | 0.0011 g |
| nickel(II)-sulfate | 0.012 g |
| cobalt(II)-chloride | 0.97 g |
| ethylenediaminetetraacetic acid salt (EDTA Na₂.2H₂O) | 0.011 g |
| L-(+)-ascorbic acid | 0.012 g |
| iron(II)-sulfate | 5.76 g |
| magnesium sulfate | 0.206 g |
| succinic acid | 0.011 g |
| manganese(II)-sulfate | 0.021 g |
| L-(+)-tartaric acid | 0.011 g |
| boric acid | 0.011 g |
| glycerol (87 %) | 0.21 g |
| potassium sodium tartrate | 0.012 g |

Further the results of tests and examinations carried out for determining the pharmaceutical activity of the composition of the invention are described in detail.

The tests and examinations were carried out in several institutes under the control of a doctor.

The patients being the subjects of the tests were male and female of different age. They received the composition according to the invention in solution form (1 ml corresponds to about 18 drops). The drops comprising the composition were administered in tea or soft drink. In addition to the drops, the patients were also administered 100 to 300 mg of ascorbic acid per day in the form of tablets or an aqueous solution, depending on the number of drops received. The patients suffering from hyperacidity were optionally administered acid, too.

### A) Test of pain-killer activity in case of chronic pains deriving from degenerative locomotor disorders

Method: random, double-blind, with placebo control.

Place of the test: Orthopedic Clinic of the Semmelweis Medical University, Budapest.

The composition of Example 1 was administered to adult patients in the following doses:
- the patients having a weight of more than 70 kg received 3 x 20 drops daily for 1 week, then 2 x 20 drops for 2 weeks and - depending on the improvement - 2 x 10 or 1 x 10 drops daily for further 2 weeks,
- the patients having a weight of less than 70 kg received 2 x 20 drops daily for 1 week, then 2 x 20 drops for 2 weeks and - depending on the improvement - 1 x 10
drops daily for further 1 or 2 weeks.

The test covered 156 patients from which 76 patients (Group I) received effective compostion, while 86 patients (Group II) were administered placebo. Improvement was observed in Group I with 57 patients (75 %) and in Group II with 26 patients (32.5 %). In Groups I and II the condition of 19 (25 %) and 54 (67.5 %) patients, respectively, remained constant.

The test results classified according to the specific disorders of the patients are summarized in Table 1.

**Table 1**

| Diagnosis | Number of patients | | |
|---|---|---|---|
| | total | improved (%) | unchanged (%) |
| Periarthritis humeroscapularis | 5 | 5 (100) | 0 (0) |
| Osteoporosis | 8 | 7 (87.5) | 1 (12.5) |
| Spondylosis dors. et lumb. | 19 | 15 (79) | 4 (21) |
| Arthrosis genus | 12 | 9 (75) | 3 (25) |
| Lumbago, lumboischialgia, stenosis canalis spinalis | 3 | 2 (67) | 1 (33) |
| Other: arthrosis cubiti, arthrosis radiocarp., osteomyelitis, PcP, necr.cap. fem. | 13 | 10 (77) | 3 (23) |

### B) Testing of pain-killer activity in case of chronic pain syndromes accompanying tumorous diseases

Method: randomised, with morphine control.

The tests were carried out in the State Oncological Clinic, Budapest.

35 patients were involved in the test, from which 17 patients received morphine and 1 drop of the composition of Example 2, calculated for 1 kg of body weight, at each treatment (Group I), while 18 patients were administered only morphine (Group II). In Group I the dose of morphine was reduced to the half of the dose received by the patients of Group II.

Total pain easement could be achieved in the case of 12 patients (71 %) in Group I, while the same could be attained by the administration of double morphine doses with 11 (61 %) patients in Group II. Reduction but not easing of pain was observed in Groups I and II in case of 5 (29 %) and 7 (39 %) patients, respectively.

### C) Examination of changes of clinical condition of children suffering from mucoviscidosis

Mucoviscidosis is a congenital illness based on genetic lesion and is considered as an incurable disease even in our days. Its most conspicuous manifestation is that the product of the glands becomes highly viscous, contrary to the product of the normal glands. Therefore, the thick discharge produced by the glands adhere to the surface of the lungs and respiratory tracks covered by mucous membrane, it cannot be discharged spontaneously. The accumulated discharge and the pathogens dwelling in it will result in permanent inflammation which can lead to the destruction of the lungs. In the digestive tract the most severe consequence is the insufficient enzyme level in the duodenum due to the condensed pancreatic fluid which results in decreased digestion and utilization of the nutritive substances. As a result, the patients have abundant, undigested stool and still suffer from chronic lack of energy.

The lifetime treatment is focused on the dissolution of the thick discharge and the substitution of the missing enzymes and vitamins. Nevertheless, not all insufficiencies can be solved completely even in spite of the most careful treatment. In addition, the susceptibility to infections is also higher.

The test was started on 40 children suffering from mucoviscidosis, from which 5 children gave up the taking of the composition of the invention. Thus, the results relate to the treatment of only 35 children. The age of the children varied between 3 and 18 years.

Method: the composition according to Example 3 was administered for 6 months in a daily dose of 1 drop per kg body weight.

The test was carried out in the Heim Pal Children's Hospital, Budapest.

As a result of the treatment with the composition of the invention, the general state of health and physical activity of a significant part of the children improved. School-children could better tolerate the school-related loads. The appetite of a great part of the children improved and, as a consequence, their body weight increased.

The change of the iron and zinc content in the blood serum of the patients was measured and it was found that the zinc and iron levels increased in the case of 32 (91 %) and 18 (51 %) patients, respectively, and played with high probability a significant role in the improvement of the patients. An other favourable result was that also the ferritin content increased in the blood serum of 25 (71 %) patients.

The observations relating to the clinical condition of the patients are comprised in Table 2.

**Table 2**

| | Improved No. of % patients | Unchanged No. of % patiens | |
|---|---|---|---|
| Body weight | 28 (80) | 7 (20) | |
| Height | 30 (86) | 5 (14) | |

| | | A % | B % |
|---|---|---|---|
| State of health | 27 (77) | 5 (14) | 3 (9) |
| Physical activity | 24 (69) | 6 (17) | 5 (14) |
| Appetite | 28 (80) | 4 (11) | 3 (9) |
| Stool | 15 (43) | 10 (54) | 1 (3) |

| | | | |
|---|---|---|---|
| A = the feature was favourable previously, too | | | |
| B = the feature was unfavourable previously | | | |

The above tests unambiguously prove that the composition according to the invention ensures very favourable clinical results in the treatment of mucoviscidosis and chronic pain syndromes deriving from degenerative locomotor diseases or accompanying diseases of tumorous origin.

The dose of the pharmaceutical composition of the invention depends on the state, body weight and illness of the patient. The daily dose may vary between 5 and 500 drops, and it amounts suitably to 20 to 150 drops, preferably to 40 to 80 drops per day.

## Claims (Claims for the following Contracting State(s): AT, BE, CH, DE, DK, FR, GB, IT, LI, LU, MC, NL, PT, SE)

1. A pharmaceutical composition having a pH of 1.9 to 4.0 suitable for influencing the reticuloendothelic system and for treating mucoviscidosis and chronic pain syndromes deriving from degenerative locomotor diseases or accompanying the diseases of tumorous origin, containing
i) one or more pharmaceutically acceptable, water-soluble compounds of boron, fluorine, magnesium, vanadium, manganese, iron, cobalt, nickel, copper, zinc and molybdenum, which compounds do not precipitate with each other or with the other components of the composition and exhibit a neutral or acidic pH in an aqueous medium,
ii) glycine,
iii) glycerol,
iv) L-(+)-ascorbic acid,
v) a neutral or acidic and water-soluble, pharmaceutically acceptable salt of ethylenediaminetetraacetic acid,
vi) potassium sodium tartrate
and
vii) succinic acid
and
viii) L-(+)-tartaric acid,
optionally together with one or more usual pharmaceutical carrier(s), diluent(s) and/or auxiliary agent(s).

2. A composition as claimed in claim 1, characterized in that it contains the boron compound(s) [i)] : fluorine compound(s) [i)] : magnesium compound(s) [i)] : vanadium compound(s) [i)] : manganese compound(s) [i)] : iron compound(s) [i)] : cobalt compound(s) [i)] : nickel compound(s) [i)] : copper compound(s) [i)] : zinc compound(s) [i)] : molybdenum compound(s) [i)]: glycine [ii)] : glycerol [iii)] : L-(+)-ascorbic acid [iv)] : ethylenediaminetetraacetic acid salt [v)] : potassium sodium tartrate [vi)] : succinic acid [vii)] : L-(+)--tartaric acid [viii)] in a weight ratio of 0.01 to 1 : 0.01 to 1 : 0.2 to 3 : 0.001 to 0.6 : 0.02 to 2 : 0.15 to 6 : 0.002 to 1 : 0.01 to 2 : 0.01 to 1 : 0.1 to 3 : 0.001 to 0.8 : 0.1 to 2 : 0.2 to 8 : 0.01 to 2 : 0.01 to 3 : 0.01 to 10 : 0.001 to 2 : 0.1 to 2.

3. A composition as claimed in claim 1 or 2, characterized in that it contains boric acid as boron compound [i)].

4. A composition as claimed in claims 1 to 3, characterized in that it contains sodium fluoride and/or vanadium trifluoride as fluorine compound(s) [i)].

5. A composition as claimed in claims 1 to 4, characterized in that it contains magnesium sulfate and/or magnesium chloride and/or [a] hydrate(s) thereof as magnesium compound(s) [i)].

6. A composition as claimed in claims 1 to 5, characterized in that it contains ammonium vanadate and/or vanadium trifluoride as vanadium compound(s) [i)].

7. A composition as claimed in claims 1 to 6, characterized in that it contains manganese sulfate and/or manganese chloride and/or [a] hydrate(s) thereof as manganese compound(s) [i)].

8. A composition as claimed in claims 1 to 7, characterized in that it contains iron(II)-sulfate and/or iron(III)-sulfate and/or [a] hydrate(s) thereof as iron compound(s) [i)].

9. A composition as claimed in claims 1 to 8, characterized in that it contains cobalt chloride and/or cobalt sulfate and/or [a] hydrate(s) thereof as cobalt compound(s) [i)].

10. A composition as claimed in claims 1 to 9, characterized in that it contains nickel chloride and/or nickel sulfate and/or [a] hydrate(s) thereof as nickel compound(s) [i)].

11. A composition as claimed in claims 1 to 10, characterized in that it contains copper(II)-sulfate or [a] hydrate(s) thereof as copper compound(s) [i)].

12. A composition as claimed in claims 1 to 11, characterized in that it contains zinc sulfate and/or [a] hydrate(s) thereof as zinc compound(s) [i)].

13. A composition as claimed in claims 1 to 12, characterized in that it contains ammonium molybdate and/or sodium molybdate as molybdenum compound(s) [i)].

14. A composition as claimed in claims 1 to 13, characterized in that it contains the disodium salt of ethylenediaminetetraacetic acid and/or [a] hydrate(s) thereof as ethylenediaminetetraacetic acid salt [v)].

15. A composition as claimed in claims 1 to 14, characterized in that it contains as substance(s) for adjusting the pH of the composition to 1.9 to 4.0 one or more pharmaceutically acceptable acid(s) in an amount necessary therefor.

16. A composition as claimed in claims 1 to 15, characterized in that it is in a form suitable for oral, rectal or vaginal administration.

17. A composition as claimed in claims 1 to 16, characterized in that it contains the boron compound(s) [i)] in an amount of 0.01 to 1.0% by weight/volume, calculated on the total volume of the composition.

18. A composition as claimed in claims 1 to 17, characterized in that it contains the fluorine compound(s) [i)] in an amount of 0.01 to 1.0% by weight/volume, calculated on the total volume of the composition.

19. A composition as claimed in claims 1 to 18, characterized in that it contains the magnesium compound(s) [i)] in an amount of 0.2 to 3.0% by weight/volume, calculated on the total volume of the composition.

20. A composition as claimed in claims 1 to 19, characterized in that it contains the vanadium compound(s) [i)] in an amount of 0.001 to 0.6% by weight/volume, calculated on the total volume of the composition.

21. A composition as claimed in claims 1 to 20, characterized in that it contains the manganese compound(s) [i)] in an amount of 0.02 to 2.0% by weight/volume, calculated on the total volume of the composition.

22. A composition as claimed in claims 1 to 21, characterized in that it contains the iron compound(s) [i)] in an amount of 0.15 to 6.0% by weight/volume, calculated on the total volume of the composition.

23. A composition as claimed in claims 1 to 22, characterized in that it contains the cobalt compound(s) [i)] in an amount of 0.002 to 1.0% by weight/volume, calculated on the total volume of the composition.

24. A composition as claimed in claims 1 to 23, characterized in that it contains the nickel compound(s) [i)] in an amount of 0.01 to 2.0% by weight/volume, calculated on the total volume of the composition.

25. A composition as claimed in claims 1 to 24, characterized in that it contains the copper compound(s) [i)] in an amount of 0.01 to 1.0% by weight/volume, calculated on the total volume of the composition.

26. A composition as claimed in claims 1 to 25, characterized in that it contains the zinc compound(s) [i)] in an amount of 0.10 to 3.0% by weight/volume, calculated on the total volume of the composition.

27. A composition as claimed in claims 1 to 26, characterized in that it contains the molybdenum compound(s) [i)] in an amount of 0.001 to 0.8% by weight/volume, calculated on the total volume of the composition.

28. A product containing a composition according to claims 1 to 27 and additionally L-(+)-ascorbic acid as a combined preparation for simultaneous, separate or sequential use in the therapy of mucoviscidosis and chronic pain syndromes deriving from locomotor diseases or accompanying the diseases of tumorous origin.

29. A product containing a composition according to claims 1 to 27 or containing a product according to claim 28 and one or more known analgesic and/or narcotic substance(s) and/or composition(s) as a combined preparation for simultaneous, separate or sequential use in the therapy of chronic pain syndromes deriving from locomotor diseases or accompanying the diseases of tumorous origin.

30. A process for preparing the pharmaceutical composition as claimed in claims 1 to 27 or the product according to claim 28 or 29, characterized by reacting the substances i) to viii) in aqueous medium and then optionally mixing the solution thus obtained with an additional amount of L-(+)-ascorbic acid and/or one or more analgesic and/or narcotic substance(s) and/or composition(s) and/or with one or more pharmaceutically acceptable carrier (s), diluent(s) and/or excipient(s) and forming a pharmaceutical composition.

## Claims (Claims for the following Contracting State(s): ES, GR)

1. A process for preparing a pharmaceutical composition having a pH of 1.9 to 4.0 suitable for influencing the reticuloendothelic system and for treating mucoviscidosis and chronic pain syndromes deriving from degenerative locomotor diseases or accompanying the diseases of tumorous origin, characterized by reacting the substances
i) one or more pharmaceutically acceptable, water-soluble compounds of boron, fluorine, magnesium, vanadium, manganese, iron, cobalt, nickel, copper, zinc and molybdenum, which compounds do not precipitate with each other or with the other components of the composition and exhibit a neutral or acidic pH in an aqueous medium,
ii) glycine,
iii) glycerol,
iv) L-(+)-ascorbic acid,
v) a neutral or acidic and water-soluble, pharmaceutically acceptable salt of ethylenediaminetetraacetic acid,
vi) potassium sodium tartrate,
vii) succinic acid
and
viii) L-(+)-tartaric acid,
optionally together with one or more usual pharmaceutical carrier(s), diluent(s) and/or auxiliary agent(s), in aqueous medium and then optionally mixing the solution thus obtained with an additional amount of L-(+)-ascorbic acid and/or one or more analgesic and/or narcotic substance(s) and/or composition(s) and/or with one or more pharmaceutically acceptable carrier(s), diluent(s) and/or excipient(s) and forming a pharmaceutical composition.

2. The process as claimed in claim 1, characterized in that the composition contains the boron compound(s) [i)] : fluorine compound(s) [i)] : magnesium compound(s) [i)] : vanadium compound(s) [i)] : manganese compound(s) [i)] : iron compound(s) [i)] : cobalt compound(s) [i)] : nickel compound(s) [i)] : copper compound(s) [i)] : zinc compound(s) [i)] : molybdenum compound(s) [i)] : glycine [ii)] : glycerol [iii)] : L-(+)-ascorbic acid [iv)] : ethylenediaminetetraacetic acid salt [v)] : potassium sodium tartrate [vi)] : succinic acid [vii)] : L-(+)-tartaric acid [viii)] in a weight ratio of 0.01 to 1 : 0.01 to 1 : 0.2 to 3 : 0.01 to 0.6 : 0.02 to 2 : 0.15 to 6 : 0.002 to 1 : 0.01 to 2 : 0.01 to 1 : 0.1 to 3 : 0.001 to 0.8 : 0.1 to 2 : 0.2 to 8 : 0.01 to 2 : 0.01 to 3 : 0.01 to 10 : 0.001 to 2 : 0.1 to 2.

3. The process as claimed in claim 1 or 2, characterized in that the composition contains boric acid as boron compound [i)].

4. The process as claimed in claims 1 to 3, characterized in that the composition contains sodium fluoride and/or vanadium trifluoride as fluorine compound(s) [i)].

5. The process as claimed in claims 1 to 4, characterized in that the composition contains magnesium sulfate and/or magnesium chloride and/or [a] hydrate(s) thereof as magnesium compound(s) [i)].

6. The process as claimed in claims 1 to 5, characterized in that the composition contains ammonium vanadate and/or vanadium trifluoride as vanadium compound(s) [i)].

7. The process as claimed in claims 1 to 6, characterized in that the composition contains manganese sulfate and/or manganese chloride and/or [a] hydrate(s) thereof as manganese compound(s) [i)].

8. The process as claimed in claims 1 to 7, characterized in that the composition contains iron(II)-sulfate and/or iron(III)-sulfate and/or [a] hydrate(s) thereof as iron compound(s) [i)].

9. The process as claimed in claims 1 to 8, characterized in that the composition contains cobalt chloride and/or cobalt sulfate and/or [a] hydrate(s) thereof as cobalt compound(s) [i)].

10. The process as claimed in claims 1 to 9, characterized in that the composition contains nickel chloride and/or nickel sulfate and/or [a] hydrate(s) thereof as nickel compound(s) [i)].

11. The process as claimed in claims 1 to 10, characterized in that the composition contains copper(II)-sulfate or [a] hydrate(s) thereof as copper compound(s) [i)].

12. The process as claimed in claims 1 to 11, characterized in that the composition contains zinc sulfate and/or [a] hydrate(s) thereof as zinc compound(s) [i)].

13. The process as claimed in claims 1 to 12, characterized in that the composition contains ammonium molybdate and/or sodium molybdate as molybdenum compound(s) [i)].

14. The process as claimed in claims 1 to 13, characterized in that the composition contains the disodium salt of ethylenediaminetetraacetic acid and/or [a] hydrate(s) thereof as ethylenediaminetetraacetic acid salt [v)].

15. The process as claimed in claims 1 to 14, characterized in that the composition contains as substance(s) for adjusting the pH of the composition to 1.9 to 4.0 one or more pharmaceutically acceptable acid(s) in an amount necessary therefor.

16. The process as claimed in claims 1 to 15, characterized in that it is in a form suitable for oral, rectal or vaginal administration.

17. The process as claimed in claims 1 to 16, characterized in that the composition contains the boron compound(s) [i)] in an amount of 0.01 to 1.0% by weight/volume, calculated on the total volume of the composition.

18. The process as claimed in claims 1 to 17, characterized in that the composition contains the fluorine compound(s) [i)] in an amount of 0.01 to 1.0% by weight/volume, calculated on the total volume of the composition.

19. The process as claimed in claims 1 to 18, characterized in that the composition contains the magnesium compound(s) [i)] in an amount of 0.2 to 3.0% by weight/volume, calculated on the total volume of the composition.

20. The process as claimed in claims 1 to 19, characterized in that the composition contains the vanadium compound(s) [i)] in an amount of 0.001 to 0.6% by weight/volume, calculated on the total volume of the composition.

21. The process as claimed in claims 1 to 20, characterized in that the composition contains the manganese compound(s) [i)] in an amount of 0.02 to 2.0% by weight/volume, calculated on the total volume of the composition.

22. The process as claimed in claims 1 to 21, characterized in that the composition contains the iron compound(s) [i)] in an amount of 0.15 to 6.0% by weight/volume, calculated on the total volume of the composition.

23. The process as claimed in claims 1 to 22, characterized in that the composition contains the cobalt compound(s) [i)] in an amount of 0.002 to 1.0% by weight/volume, calculated on the total volume of the composition.

24. The process as claimed in claims 1 to 23, characterized in that the composition contains the nickel compound(s) [i)] in an amount of 0.01 to 2.0% by weight/volume, calculated on the total volume of the composition.

25. The process as claimed in claims 1 to 24, characterized in that the composition contains the copper compound(s) [i)] in an amount of 0.01 to 1.0% by weight/volume, calculated on the total volume of the composition.

26. The process as claimed in claims 1 to 25, characterized in that the composition contains the zinc compound(s) [i)] in an amount of 0.10 to 3.0% by weight/volume, calculated on the total volume of the composition.

27. The process as claimed in claims 1 to 26, characterized in that the composition contains the molybdenum compound(s) [i)] in an amount of 0.001 to 0.8% by weight/volume, calculated on the total volume of the composition.

28. A process for preparing a product containing a composition prepared according to claims 1 to 27 and additionally L-(+)-ascorbic acid as a combined preparation for simultaneous, separate or sequential use in the therapy of mucoviscidosis and chronic pain syndromes deriving from locomotor diseases or accompanying the diseases of tumorous origin, characterized by reacting the substances i) to viii) in aqueous medium and then optionally mixing the solution thus obtained with an additional amount of L-(+)-ascorbic acid and/or one or more analgesic and/or narcotic substance(s) and/or composition(s) and/or with one or more pharmaceutically acceptable carrier(s), diluent(s) and/or excipient(s) and forming a pharmaceutical composition.

29. A process for preparing a product containing a composition prepared according to claims 1 to 27 or containing a product prepared according to claim 28 and one or more known analgesic and/or narcotic substance(s) and/or composition(s) as a combined preparation for simultaneous, separate or sequential use in the therapy of chronic pain syndromes deriving from locomotor diseases or accompanying the diseases of tumorous origin, characterized by reacting the substances i) to viii) in aqueous medium and then optionally mixing the solution thus obtained with an additional amount of L-(+)-ascorbic acid and/or one or more analgesic and/or narcotic substance(s) and/or composition(s) and/or with one or more pharmaceutically acceptable carrier(s), diluent(s) and/or excipient(s) and forming a pharmaceutical composition.

## Patentansprüche (Patentansprüche für folgende(n) Vertragsstaat(en): AT, BE, CH, DE, DK, FR, GB, IT, LI, LU, MC, NL, PT, SE)

1. Eine pharmazeutische Komposition mit einem pH von 1,9 bis 4,0, die fähig ist zur Beeinflussung des retikuloendothelischen Systems, zur Behandlung von Mucoviscidosis und chronischen Schmerzsyndromen, die von degenerativen lokomotorischen Krankheiten stammen oder Krankheiten tumorischen Ursprungs begleiten, enthaltend
i) eine oder mehrere pharmazeutisch annehmbare, wasserlösliche Verbindungen von Bor, Fluor, Magnesium, Vanadium, Mangan, Eisen, Kobalt, Nickel, Kupfer, Zink und Molybden, wobei diese Verbindungen mit einander oder mit den anderen Komponenten der Komposition keinen Niederschlag bilden und in wässrigem Medium einen neutralen oder sauren pH aufweisen,
ii) Glycin,
iii) Glycerin,
iv) L-(+)-Ascorbinsäure,
v) ein neutrales oder saures und wasserlösliches, pharmazeutisch annehmbares Salz der Ethylendiamintetraessigsäure,
vi) Kaliumnatrium-tartarat und
vii) Bernsteinsäure und
viii) L-(+)-Weinsteinsäure,
gegebenenfalls zusammen mit einem oder mehreren üblichen pharmazeutischen Trägerstoff(en), Verdünnungsmittel(n) und/oder Hilfsmittel(n).

2. Eine Komposition nach Anspruch 1, dadurch gekennzeichnet, dass sie die Borverbindung(en) [i)] : Fluorverbindung(en)] [i)] : Magnesiumverbindung(en) [i)] : Vanadiumverbindung(en) [i)] : Manganverbindung(en) [i)] : Eisenverbindung(en) [i)] : Kobaltverbindung(en) [i)] : Nickelverbindung(en) [i)] : Kupferverbindung(en) [i)] : Zinkverbindung(en) [i)] : Molybdenverbindung(en) [i)] : Glycin, [ii)] : Glycerin, [iii)] : L-(+)Ascorbinsäure [iv)] : Ethylendiamintetraessigsäure v)] : Kaliumnatrium-tartarat (vi)] : Bernsteinsäure (vii)] : L-(+)-Weinsteinsäure (viii)] in einem Gewichtsverhältnis von 0,01 bis 1 : 0,01 bis 1 : 0,2 bis 3 : 0,01 bis 0,6 : 0,02 bis 2 : 0,15 bis 6 : 0,02 bis 1 : 0,01 bis 2 : 0,01 bis 1 : 0,1 bis 3 : 0,001 bis 0,8 : 0,1 bis 2 : 0,2 bis 8 : 0,01 bis 2 : 0,01 bis 3 : 0,01 bis 10 : 0,001 bis 2 : 0,1 bis 2 enthält.

3. Eine Komposition nach Patentanspruch 1 oder 2, dadurch gekennzeichnet, dass sie Borsäure als Borverbindung [i)] enthält.

4. Eine Verbindung nach den Patentansprüchen 1 bis 3, dadurch gekennzeichnet, dass sie Natriumfluorid und/oder Vanadiumtrifluorid als Fluorverbindung(en) [i)] enthält.

5. Eine Komposition nach den Patentansprüchen 1 bis 4, dadurch gekennzeichnet, dass sie Magnesiumsulfat und/oder Magnesiumchlorid und/oder (ein) Hydrat(e) davon als Magnesiumverbindung(en) [i)] enthält.

6. Eine Komposition nach den Patentansprüchen 1 bis 5, dadurch gekennzeichnet, dass sie Ammoniumvanadat und/oder Vanadiumtrifluorid als Vanadiumverbindung(en) [i)] enthält.

7. Eine Komposition nach den Patentansprüchen 1 bis 6, dadurch gekennzeichnet, dass sie Mangansulfat und/oder Manganchlorid und/oder (ein) Hydrat(e) davon als Manganverbindung(en) [i)] enthält.

8. Eine Komposition nach den Patentansprüchen 1 bis 7, dadurch gekennzeichnet, dass sie Eisen(II)-sulfat und/oder Eisen(III)-sulfat und/oder (ein) Hydrat(e) davon als Eisenverbindung(en) [i)] enhält.

9. Eine Komposition nach den Patentansprüchen 1 bis 8, dadurch gekennzeichnet, dass sie Kobaltchlorid und/oder Kobaltsulfat und/oder (ein) Hydrat(e) davon als Kobaltverbindung(en) [i)] enthält.

10. Eine Komposition nach den Patentansprüchen 1 bis 9, dadurch gekennzeichnet, dass sie Nickelchlorid und/oder Nickelsulfat und/oder (ein) Hydrat(e) davon als Nickelverbindung(en) [i)] enthält.

11. Eine Komposition nach den Patentansprüchen 1 bis 10, dadurch gekennzeichnet, dass sie Kupfer(II)-sulfat oder (ein) Hydrat(e) davon als Kupferverbindung(en) [i)] enthält.

12. Eine Komposition nach den Patentansprüchen 1 bis 11, dadurch gekennzeichnet, dass sie Zinksulphat und/oder (ein) Hydrat(e) davon als Zinkverbindung(en) [i)] enthält.

13. Eine Komposition nach den Patentansprüchen 1 bis 12, dadurch gekennzeichnet, dass sie Ammoniummolybdat und/oder Natriummolybdat als Molybdenverbindung(en) (i)] enthält.

14. Eine Komposition nach den Patentansprüchen 1 bis 13, dadurch gekennzeichnet, dass sie Dinatriumsalz der Ethylendiamintetraessigsäure und/oder (ein) Hydrat(e) davon als Ethylendiamintetraessigsäuresalz [v)] enthält.

15. Eine Komposition nach den Patentansprüchen 1 bis 14, dadurch gekennzeichnet, dass sie als Substanz(en) für Einstellung des pH der Komposition auf 1,9 bis 4,0 eine oder mehrere pharmazeutisch annehmbare Säure(n) in einer dafür nötigen Menge enthält.

16. Eine Komposition nach den Patentansprüchen 1 bis 15, dadurch gekennzeichnet, dass sie in einer zur oralen, rektalen oder vaginalen Verabreichung geeigneter Form vorliegt.

17. Eine Komposition nach den Patentansprüchen 1 bis 16, dadurch gekennzeichnet, dass sie die Borverbindung(en) [i)] in einer Menge von 0,01 bis 1,0 Gewichts/Volumen%, berechnet auf das totale Volumen der Komposition, enthält.

18. Eine Komposition nach den Patentansprüchen 1 bis 17, dadurch gekennzeichnet, dass sie die Fuorverbindung(en) [i)] in einer Menge von 0,01 bis 1,0 Gewichts/Volumen%, berechnet auf das totale Volumen der Komposition, enthält.

19. Eine Komposition nach den Patentansprüchen 1 bis 18, dadurch gekennzeichnet, dass sie die Magnesiumverbindung(en) [i)] in einer Menge von 0,2 bis 3,0 Gewichts/Volumen%, berechnet auf das totale Volumen der Komposition, enthält.

20. Eine Komposition nach den Patentansprüchen 1 bis 19, dadurch gekennzeichnet, dass sie die Vanadiumverbindung(en) [i)] in einer Menge von 0,001 bis 0,6 Gewichts/Volumen%, berechnet auf das totale Volumen der Komposition, enthält.

21. Eine Komposition nach den Patentansprüchen 1 bis 20, dadurch gekennzeichnet, dass sie die Manganverbindung(en) [i)] in einer Menge von 0,02 bis 2,0 Gewichts/Volumen%, berechnet auf das totale Volumen der Komposition, enthält.

22. Eine Komposition nach den Patentansprüchen 1 bis 21, dadurch gekennzeichnet, dass sie die Eisenverbindung(en) [i)] in einer Menge von 0,15 bis 6,0 Gewichts/Volumen%, berechnet auf das totale Volumen der Komposition, enthält.

23. Eine Komposition nach den Patentansprüchen 1 bis 22, dadurch gekennzeichnet, dass sie die Kobaltverbindung(en) [i)] in einer Menge von 0,002 bis 1,0 Gewichts/Volumen%, berechnet auf das totale Volumen der Komposition, enthält.

24. Eine Komposition nach den Patentansprüchen 1 bis 23, dadurch gekennzeichnet, dass sie die Nickelverbindung(en) [i)] in einer Menge von 0,01 bis 2,0 Gewichts/Volumen%, berechnet auf das totale Volumen der Komposition, enthält.

25. Eine Komposition nach den Patentansprüchen 1 bis 24, dadurch gekennzeichnet, dass sie die Kupferverbindung(en) [i)] in einer Menge von 0,01 bis 1,0 Gewichts/Volumen%, berechnet auf das totale Volumen der Komposition, enthält.

26. Eine Komposition nach den Patentansprüchen 1 bis 25, dadurch gekennzeichnet, dass sie die Zinkverbindung(en) [i)] in einer Menge von 0,10 bis 3,0 Gewichts/Volumen%, berechnet auf das totale Volumen der Komposition, enthält.

27. Eine Komposition nach den Patentansprüchen 1 bis 26, dadurch gekennzeichnet, dass sie die Molybdenverbindung(en) [i)] in einer Menge von 0,001 bis 0,8 Gewichts/Volumen%, berechnet auf das totale Volumen der Komposition, enthält.

28. Ein Produkt enthaltend eine Komposition gemäss den Patentansprüchen 1 bis 27 und zusätzlich L-(+)-Ascorbinsäure als ein kombiniertes Präparat für die gleichzeitige, separate oder nacheinanderfolgende Verwendung in der Therapie von Mucoviscidosis und chronischen Schmerzsyndromen, die von lokomotorischen Krankheiten stammen oder Krankheiten tumorischen Urpsrungs begleiten.

29. Ein Produkt enthaltend eine Komposition gemäss den Patentansprüchen 1 bis 27 oder ein Produkt gemäss dem Patentanspruch 28 und eine oder mehrere analgesische und/oder narkotische Substanz(en) und/oder Komposition(en) als ein kombiniertes Präparat für die gleichzeitige, separate oder nacheinanderfolgende Verwendung in der Therapie von Mucoviscidosis und chronischen Schmerzsyndromen, die von lokomotorischen Krankheiten stammen oder Krankheiten tumorischen Urpsrungs begleiten.

30. Ein Verfahren zur Herstellung der pharmazeutischen Komposition gemäss den Patentansprüchen 1 bis 27 oder das Produkt gemäss den Patentansprüchen 28 oder 29, dadurch gekennzeichnet, dass die Substanzen (i) bis (viii) in wässrigem Medium umgesetzt werden und danach gegebenenfalls die derart erhaltene Lösung mit einer zusätzlichen Menge von L-(+)-Ascorbinsäure und/oder einer oder mehreren analgesischen und/oder narkotischen Substanz(en) und/oder Komposition(en) und/oder mit einem oder mehreren pharmazeutisch annehmbaren Trägerstoff(en), Verdünnungsmittel(n) und/oder Hilfsstoff(en) vermischt und eine pharmazeutische Komposition gebildet wird.

## Patentansprüche (Patentansprüche für folgende(n) Vertragsstaat(en): ES, GR)

1. Ein Verfahren zur Herstellung einer pharmazeutischen Komposition mit einem pH von 1,9 bis 4,0, die fähig ist zur Beeinflussung des retikuloendothelischen Systems, zur Behandlung von Mucoviscidosis und chronischen Schmerzsyndromen, die von degenerativen lokomotorischen Krankheiten stammen oder Krankheiten tumorischen Ursprungs begleiten, dadurch gekennzeichnet, dass
i) eine oder mehrere pharmazeutisch annehmbare, wasserlösliche Verbindungen von Bor, Fluor, Magnesium, Vanadium, Mangan, Eisen, Kobalt, Nickel, Kupfer, Zink und Molybden, wobei diese Verbindungen mit einander oder mit den anderen Komponenten der Komposition keinen Niederschlag bilden und in wässrigem Medium einen neutralen oder sauren pH aufweisen,
ii) Glycin,
iii) Glycerin,
iv) L-(+)-Ascorbinsäure,
v) ein neutrales oder saures und wasserlösliches, pharmazeutisch annehmbares Salz der Ethylendiamintetraessigsäure,
vi) Kalium-natrium-tartarat und
vii) Bernsteinsäure und
viii) L-(+)-Weinsteinsäure,
gegebenenfalls zusammen mit einem oder mehreren üblichen pharmazeutischen Trägerstoff(en), Verdünnungsmittel(n) und/oder Hilfsmittel(n) in einem wässrigen Medium umgesetzt werden und danach gegebenenfalls die derart erhaltene Lösung mit einer zusätzlichen Menge von L-(+)-Ascorbinsäure und/oder einer oder mehreren analgesischen und/oder narkotischen Substanz(en) und/oder Komposition(en) und/oder mit einem oder mehreren pharmazeutisch annehmbaren Trägerstoff(en), Verdünnungsmittel(n) und/oder Hilfsstoff(en) vermischt und eine pharmazeutische Komposition gebildet wird.

2. Das Verfahren nach dem Patentanspruch 1, dadurch gekennzeichnet, dass die Komposition die Borverbindung(en) [i)] : Fluorverbindung(en)] [i)] : Magnesiumverbindung(en) [i)] : Vanadiumverbindung(en) [i)] : Manganverbindung(en) [i)] : Eisenverbindung(en) [i)] : Kobaltverbindung(en) [i)] : Nickelverbindung(en) [i)] : Kupferverbindung(en) [i)] : Zinkverbindung(en) [i)] : Molybdenverbindung(en) [i)] : Glycin, [ii)] : Glycerin, [iii)] : L-(+)Ascorbinsäure [iv)] : Ethylendiamintetraessigsäure v)] : Kalium-natrium-tartarat (vi)] Bernsteinsäure (vii)] : L-(+)-Weinsteinsäure (viii)] in einem Gewichtsverhältnis von 0,01 bis 1 : 0,01 bis 1 : 0,2 bis 3 : 0,01 bis 0,6 : 0,02 bis 2 : 0,15 bis 6 : 0,02 bis 1 : 0,01 bis 2 : 0,01 bis 1 : 0,1 bis 3 : 0,001 bis 0,8 : 0,1 bis 2 : 0,2 bis 8 : 0,01 bis 2 : 0,01 bis 3 : 0,01 bis 10 : 0,001 bis 2 : 0,1 bis 2 enthält.

3. Das Verfahren nach dem Patentanspruch 1 oder 2, dadurch gekennzeichnet, dass die Komposition Borsäure als Borverbindung [i)] enthält.

4. Das Verfahren nach den Patentansprüchen 1 bis 3, dadurch gekennzeichnet, dass die Komposition Natriumfluorid und/oder Vanadiumtrifluorid als Fuorverbindung(en) [i)] enthält.

5. Das Verfahren nach den Patentansprüchen 1 bis 4, dadurch gekennzeichnet, dass die Komposition Magnesiumsulfat und/oder Magnesiumchlorid und/oder (ein) Hydrat(e) davon als Magnesiumverbindung(en) [i)] enthält.

6. Das Verfahren nach den Patentansprüchen 1 bis 5, dadurch gekennzeichnet, dass die Komposition Ammoniumvanadat und/oder Vanadiumtrifluorid als Vanadiumverbindung(en) [i)] enthält.

7. Das Verfahren nach den Patentansprüchen 1 bis 6, dadurch gekennzeichnet, dass die Komposition Mangansulfat und/oder Manganchlorid und/oder (ein) Hydrat(e) davon als Manganverbindung(en) [i)] enthält.

8. Das Verfahren nach den Patentansprüchen 1 bis 7, dadurch gekennzeichnet, dass die Komposition Eisen(II)-sulfat und/oder Eisen(III)-sulfat und/oder (ein) Hydrat(e) davon als Eisenverbindung(en) [i)] enhält.

9. Das Verfahren nach den Patentansprüchen 1 bis 8, dadurch gekennzeichnet, dass die Komposition Kobaltchlorid und/oder Kobaltsulfat und/oder (ein) Hydrat(e) davon als Kobaltverbindung(en) [i)] enthält.

10. Das Verfahren nach den Patentansprüchen 1 bis 9, dadurch gekennzeichnet, dass die Komposition Nickelchlorid und/oder Nickelsulfat und/oder (ein) Hydrat(e) davon als Nickelverbindung(en) [i)] enthält.

11. Das Verfahren nach den Patentansprüchen 1 bis 10, dadurch gekennzeichnet, dass die Komposition sie Kupfer(II)-sulfat oder (ein) Hydrat(e) davon als Kupferverbindung(en) [i)] enthält.

12. Das Verfahren nach den Patentansprüchen 1 bis 11, dadurch gekennzeichnet, dass die Komposition Zinksulphat und/oder (ein) Hydrat(e) davon als Zinkverbindung(en) [i)] enthält.

13. Das Verfahren nach den Patentansprüchen 1 bis 12, dadurch gekennzeichnet, dass die Komposition Ammoniummolybdat und/oder Natriummolybdat als Molybdenverbindung(en) [i)] enthält.

14. Das Verfahren nach den Patentansprüchen 1 bis 13, dadurch gekennzeichnet, dass die Komposition Dinatriumsalz der Ethylendiamintetraessigsäure und/oder (ein) Hydrat(e) davon als Ethylendiamintetraessigsäuresalz [v)] enthält.

15. Das Verfahren nach den Patentansprüchen 1 bis 14, dadurch gekennzeichnet, dass die Komposition als Substanz(en) für Einstellung des pH der Komposition auf 1,9 bis 4,0 eine oder mehrere pharmazeutisch annehmbare Säure(n) in einer dafür nötigen Menge enthält.

16. Das Verfahren nach den Patentansprüchen 1 bis 15, dadurch gekennzeichnet, dass die Komposition in einer zur oralen, rektalen oder vaginalen Verabreichung geeigneter Form vorliegt.

17. Das Verfahren nach den Patentansprüchen 1 bis 16, dadurch gekennzeichnet, dass die Komposition die Borverbindung(en) [i)] in einer Menge von 0,01 bis 1,0 Gewichts/Volumen%, berechnet auf das totale Volumen der Komposition, enthält.

18. Das Verfahren nach den Patentansprüchen 1 bis 17, dadurch gekennzeichnet, dass die Komposition die Fluorverbindung(en) [i)] in einer Menge von 0,01 bis 1,0 Gewichts/Volumen%, berechnet auf das totale Volumen der Komposition, enthält.

19. Das Verfahren nach den Patentansprüchen 1 bis 18, dadurch gekennzeichnet, dass die Komposition die Magnesiumverbindung(en) [i)] in einer Menge von 0,2 bis 3,0 Gewichts/Volumen%, berechnet auf das totale Volumen der Komposition, enthält.

20. Das Verfahren nach den Patentansprüchen 1 bis 19, dadurch gekennzeichnet, dass die Komposition die Vanadiumverbindung(en) [i)] in einer Menge von 0,001 bis 0,6 Gewichts/Volumen%, berechnet auf das totale Volumen der Komposition, enthält.

21. Das Verfahren nach den Patentansprüchen 1 bis 20, dadurch gekennzeichnet, dass die Komposition die Manganverbindung(en) [i)] in einer Menge von 0,02 bis 2,0 Gewichts/Volumen%, berechnet auf das totale Volumen der Komposition, enthält.

22. Das Verfahren nach den Patentansprüchen 1 bis 21, dadurch gekennzeichnet, dass die Komposition die Eisenverbindung(en) [i)] in einer Menge von 0,15 bis 6,0 Gewichts/Volumen%, berechnet auf das totale Volumen der Komposition, enthält.

23. Das Verfahren nach den Patentansprüchen 1 bis 22, dadurch gekennzeichnet, dass die Komposition die Kobaltverbindung(en) [i)] in einer Menge von 0,002 bis 1,0 Gewichts/Volumen%, berechnet auf das totale Volumen der Komposition, enthält.

24. Das Verfahren nach den Patentansprüchen 1 bis 23, dadurch gekennzeichnet, dass die Komposition die Nickelverbindung(en) [i)] in einer Menge von 0,01 bis 2,0 Gewichts/Volumen%, berechnet auf das totale Volumen der Komposition, enthält.

25. Das Verfahren nach den Patentansprüchen 1 bis 24, dadurch gekennzeichnet, dass die Komposition die Kupferverbindung(en) [i)] in einer Menge von 0,01 bis 1,0 Gewichts/Volumen%, berechnet auf das totale Volumen der Komposition, enthält.

26. Das Verfahren nach den Patentansprüchen 1 bis 25, dadurch gekennzeichnet, dass die Komposition die Zinkverbindung(en) [i)] in einer Menge von 0,10 bis 3,0 Gewichts/Volumen%, berechnet auf das totale Volumen der Komposition, enthält.

27. Das Verfahren nach den Patentansprüchen 1 bis 26, dadurch gekennzeichnet, dass die Komposition die Molybdenverbindung(en) [i)] in einer Menge von 0,001 bis 0,8 Gewichts/Volumen%, berechnet auf das totale Volumen der Komposition, enthält.

28. Ein Verfahren zur Herstellung eines Produktes, das als ein kombiniertes Präparat eine gemäss den Patentansprüchen 1 bis 27 hergestellte Komposition und zusätzlich L-(+)-Ascorbinsäure enthält, für die gleichzeitige, separate oder nacheinanderfolgende Verwendung in der Therapie von Mucoviscidosis und chronischen Schmerzsyndromen, die von lokomotorischen Krankheiten stammen oder Krankheiten tumorischen Ursprungs begleiten, dadurch gekennzeichnet, dass die Substanzen (i) bis (viii) in wässrigem Medium umgesetzt werden und danach gegebenenfalls die derart erhaltene Lösung mit einer zusätzlichen Menge von L-(+)-Ascorbinsäure und/oder einer oder mehreren analgesischen und/oder narkotischen Substanz(en) und/oder Komposition(en) und/oder mit einem oder mehreren pharmazeutisch annehmbaren Trägerstoff(en), Verdünnungsmittel(n) und/oder Hilfsstoff(en) vermischt und eine pharmazeutische Komposition gebildet wird.

29. Ein Verfahren zur Herstellung eines Produktes, das eine gemäss den Patentansprüchen 1 bis 27 hergestellte Komposition oder ein gemäss dem Patentanspruch 28 hergestelltes Produkt und eine oder mehrere bekannte analgesische und/oder narkotische Substanz(en) und/oder Komposition(en) als ein kombiniertes Präparat enthält, für die gleichzeitige, separate oder nacheinanderfolgende Verwendung in der Therapie von chronischen Schmerzsyndromen, die von lokomotorischen Krankheiten stammen oder Krankheiten tumorischen Ursprungs begleiten, dadurch gekennzeichnet; dass die Substanzen (i) bis (viii) in wässrigem Medium umgesetzt werden und danach gegebenenfalls die derart erhaltene Lösung mit einer zusätzlichen Menge von L-(+)-Ascorbinsäure und/oder einer oder mehreren analgesischen und/oder narkotischen Substanz(en) und/oder Komposition(en) und/oder mit einem oder mehreren pharmazeutisch annehmbaren Trägerstoff(en), Verdünnungsmittel(n) und/oder Hilfsstoff(en) vermischt und eine pharmazeutische Komposition gebildet wird.

## Revendications (Revendications pour l'(les) Etat(s) contractant(s) suivant(s): AT, BE, CH, DE, DK, FR, GB, IT, LI, LU, MC, NL, PT, SE)

1. Composition pharmaceutique ayant une valeur pH de 1,9 à 4,0, propre à exercer une influence sur le système réticuloendothélique, à traiter la mucoviscidose et les syndrômes de douleur chronique dus à des maladies locomotrices dégénératives ou accompagnant des maladies d'origine tumorale, et qui contient
i) un ou plusieurs composés de bore, de fluor, de magnésium, de vanadium, de manganèse, de fer, de cobalt, de nickel, de cuivre, de zinc et de molybdène, acceptables du point de vue pharmaceutique et solubles dans l'eau, et qui ne se séparent pas par précipitation avec les autres composés de la composition, et qui ont un pH neutre ou acide en milieu aqueux,
ii) de la glycine,
iii) du glycérol,
iv) de l'acide L-(+)-ascorbique,
v) un sel neutre ou acide et soluble dans l'eau, acceptable du point de vue pharmaceutique, de l'acide éthylènediaminetétraacétique,
vi) du tartrate de sodium-potassium et
vii) et de l'acide succinique et
viii) de l'acide L(+)-tartrique,
si cela est désiré ensemble avec un ou plusieurs porteur(s), diluant(s) et/ou agent(s) auxiliaire(s) habituel(s) acceptable(s) du point de vue pharmaceutique.

2. Une composition suivant la Revendication 1, caractérisée en ce qu'elle contient le(s) composé(s) de bore [i)] : le(s) composé(s) de fluor]
[i)] : le(s) composé(s) de magnésium [i)] : le(s) composé(s) de vanadium [i)] : le(s) composé(s) de manganèse [i)] : le(s) composé(s) de fer [i)] : le(s) composé(s) de cobalt [i)] : le(s) composé(s) de nickel [i)] : le(s) composé(s) de cuivre [i)] : le(s) composé(s) de zinc [i)] : le(s) composé(s) de molybdène [i)] : de la glycine, [ii)] : du glycérol, [iii)] : de l'acide L-(+)ascorbique [iv)] : de l'acide éthylènediaminetétraacétique v)] : du tartrat de potassium-sodium (vi)] : de l'acide succinique (vii)] : de l'acide L-(+)-tartrique (viii)] dans un rapport pondéral de 0,01 à 1 : 0,01 à 1 : 0,2 à 3 : 0,01 à 0,6 : 0,02 à 2 : 0,15 à 6 : 0,02 à 1 : 0,01 à 2 : 0,01 à 1 : 0,1 à 3 : 0,001 à 0,8 : 0,1 à 2 : 0,2 à 8 : 0,01 à 2 : 0,01 à 3 : 0,01 à 10 : 0,001 à 2 : 0,1 à 2.

3. Une composition selon la Revendication 1 ou 2, caractérisée en ce qu'elle contient à titre de composé boré de l'acide borique [i)].

4. Une composition selon les Revendications 1 à 3, caractérisée en ce qu'elle contient à titre de composé fluoré [i)] du fluorure de sodium et/ou du trifluorure de vanadium.

5. Une composition selon les Revendications 1 à 4, caractérisée en ce qu'elle contient à titre de composé(s) de magnésium [i)] du sulfate de magnésium et/ou du chlorure de magnésium et/ou un (des) hydrate(s) de ceux-ci.

6. Une composition selon les Revendications 1 à 5, caractérisée en ce qu'elle contient à titre de composé de vanadium [i)] du vanadate d'ammonium et/ou du trifluorure de vanadium.

7. Une composition selon les Revendications 1 à 6, caractérisée en ce qu'elle contient à titre de composé(s) de manganèse [i)] du sulfate de manganèse et/ou du chlorure de manganèse et/ou un (des) hydrate(s) de ceux-ci.

8. Une composition selon les Revendications 1 à 7, caractérisée en ce qu'elle contient à titre de composé(s) de fer [i)] du sulfate de fer (II) et/ou du sulfate de fer(III) et/ou un (des) hydrate(s) de ceux-ci.

9. Une composition selon les Revendications 1 à 8, caractérisée en ce qu'elle contient à titre de composé(s) de cobalt [i)] du chlorure de cobalt et/ou du sulfate de cobalt et/ou un (des) hydrate(s) de ceux-ci.

10. Une composition selon les Revendications 1 à 9, caractérisée en ce qu'elle contient à titre de composé(s) de nickel [i)] du chlorure de nickel et/ou du sulfate de nickel et/ou un (des) hydrate(s) de ceux-ci.

11. Une composition selon les Revendications 1 à 10, caractérisée en ce qu'elle contient à titre de composé(s) de cuivre [i)] du sulfate de cuivre (II) ou un (des) hydrate(s) de ceux-ci.

12. Une composition selon les Revendications 1 à 11, caractérisée en ce qu'elle contient à titre de composé(s) de zinc [i)] du sulfate de zinc et/ou un (des) hydrate(s) de ceux-ci.

13. Une composition selon les Revendications 1 à 12, caractérisée en ce qu'elle contient à titre de composé(s) de molybdène [i)] du molybdate d'ammonium et/ou du molybdate de sodium.

14. Une composition selon les Revendications 1 à 13, caractérisée en ce qu'elle contient à titre de sel d'acide éthylènediaminetétraacétique [v)] du sel disodique d'acide éthylènediaminetétraacétique et/ou un (des) hydrate(s) de ceux-ci.

15. Une composition selon les Revendications 1 à 14, caractérisée en ce qu'elle contient à titre de substance(s) d'ajustement du pH de la composition sur 1,9 à 4,0, un ou plusieurs acide(s) acceptable(s) du point de vue pharmaceutique dans la quantité qui y est nécessaire.

16. Une composition selon les Revendications 1 à 15, caractérisée en ce qu'elle est disponible sous une forme permettant l'administration orale, rectale ou vaginale.

17. Une composition selon les Revendications 1 à 16, caractérisée en ce qu'elle contient le(s) composé(s) boré(s) [i)] en une quantité de 0,01 à 1,0 % poids/volume, calculée sur le poids total de la composition.

18. Une composition selon les Revendications 1 à 17, caractérisée en ce qu'elle contient le(s) composé(s) fluoré(s) [i)] en une quantité de 0,01 à 1,0 % poids/volume, calculée sur le poids total de la composition.

19. Une composition selon les Revendications 1 à 18, caractérisée en ce qu'elle contient le(s) composé(s) de magnésium [i)] en une quantité de 0,2 à 3,0 % poids/volume, calculée sur le poids total de la composition.

20. Une composition selon les Revendications 1 à 19, caractérisée en ce qu'elle contient le(s) composé(s) de vanadium [i)] en une quantité de 0,001 à 0,6 % poids/volume, calculée sur le poids total de la composition.

21. Une composition selon les Revendications 1 à 20, caractérisée en ce qu'elle contient le(s) composé(s) de manganèse [i)] en une quantité de 0,02 à 2,0 % poids/volume, calculée sur le poids total de la composition.

22. Une composition selon les Revendications 1 à 21, caractérisée en ce qu'elle contient le(s) composé(s) de fer [i)] en une quantité de 0,15 à 6,0 % poids/volume, calculée sur le poids toal de la composition.

23. Une composition selon les Revendications 1 à 22, caractérisée en ce qu'elle contient le(s) composé(s) de cobalt [i)] en une quantité de 0,002 à 1,0 % poids/volume, calculée sur le poids toal de la composition.

24. Une composition selon les Revendications 1 à 23, caractérisée en ce qu'elle contient le(s) composé(s) de nickel [i)] en une quantité de 0,01 à 2,0 % poids/volume, calculée sur le poids toal de la composition.

25. Une composition selon les Revendications 1 à 24, caractérisée en ce qu'elle contient le(s) composé(s) de cuivre [i)] en une quantité de 0,01 à 1,0 % poids/volume, calculée sur le poids toal de la composition.

26. Une composition selon les Revendications 1 à 25, caractérisée en ce qu'elle contient le(s) composé(s) de zinc [i)] en une quantité de 0,10 à 3,0 % poids/volume, calculée sur le poids total de la composition.

27. Une composition selon les Revendications 1 à 26, caractérisée en ce qu'elle contient le(s) composé(s) de molybdène [i)] en une quantité de 0,001 à 0,8 % poids/volume, calculée sur le poids total de la composition.

28. Un produit contenant une composition selon les Revendications 1 à 27 ainsi que de l'acide L-(+)-ascorbique en tant que préparation combinée destinée à un usage simultané, séparé ou successif dans la thérapie de la mucoviscidose et des syndrômes de douleur chronique dérivant de maladies locomotrices ou accompagnant des maladies d'origine tumorale.

29. Un produit contenant une composition selon les Revendications 1 à 27 ou bien un produit selon la Revendication 28 et une ou plusieurs substance(s) et/ou composition(s) analgésiques et/ou narcotiques en tant que préparation combinée destinée à un usage simultané, séparé ou successif dans la thérapie de la mucoviscidose et des syndrômes de douleur chronique dérivant de maladies locomotrices ou accompagnant des maladies d'origine tumorale.

30. Procédé pour préparer une composition pharmaceutique selon les Revendications 1 à 27 ou bien le produit selon les Revendications 28 ou 29, caractérisés en ce que l'on fait réagir les substances (i) à (viii) en mileu aqueux et que l'on mélange éventuellement la solution ainsi obtenue avec une quantité supplémentaire d'acide L-(+)-ascorbique et/ou avec une ou plusieurs substance(s) et/ou composition(s) analgésique(s) et/ou narcotique(s) et/ou avec un ou plusieurs porteur(s), diluant(s) et/ou excipient(s) acceptable(s) du point de vue pharmaceutique, et que l'on forme une composition pharmaceutique.

## Revendications (Revendications pour l'(les) Etat(s) contractant(s) suivant(s): ES, GR)

1. Procédé pour préparer une composition pharmaceutique ayant une valeur pH de 1,9 à 4,0, propre à exercer une influence sur le système réticuloendothélique, à traiter la mucoviscidose et les syndrômes de douleur chronique dus à des maladies locomotrices dégénératives ou accompagnant des maladies d'origine tumorale, caractérisé en ce que l'on fait réagir
i) un ou plusieurs composés de bore, de fluor, de magnésium, de vanadium, de manganèse, de fer, de cobalt, de nickel, de cuivre, de zinc et de molybdène, acceptables du point de vue pharmaceutique et solubles dans l'eau, et qui ne se séparent pas par précipitation avec les autres composés de la composition, et qui ont un pH neutre ou acide en milieu aqueux,
ii) de la glycine,
iii) du glycérol,
iv) de l'acide L-(+)-ascorbique,
v) un sel neutre ou acide et soluble dans l'eau, acceptable du point de vue pharmaceutique, de l'acide éthylènediaminetétra-acétique,
vi) du tartrate de sodium-potassium et
vii) et de l'acide succinique et
viii) de l'acide L-(+)-tartrique,
si cela est désiré ensemble avec un ou plusieurs porteur(s), diluant(s) et/ou agent(s) auxiliaire(s) habituel(s) acceptable(s) du point de vue pharmaceutique, en milieu aqueux et que l'on mélange éventuellement les solutions ainsi obtenues avec une quantité supplémentaire d'acide L-(+)-ascorbique et/ou d'une (de) substance(s) et/ou composition(s) analgésique(s) et/ou narcotique(s) et/ou avec un ou plusieurs porteur(s), diluant(s) et/ou excipient(s) acceptable(s) du point de vue pharmaceutique et que l'on forme une composition pharmaceutique.

2. Le procédé selon la Revendication 1, caractérisé en ce que la composition contient le(s) composé(s) boré(s) [i)] : le(s) composé(s) fluoré(s)]
[i)] : le(s) composé(s) de magnésium [i)] : le(s) composé(s) de vanadium [i)] : le(s) composé(s) de manganèse [i)] : le(s) composé(s) de fer [i)] : le(s) composé(s) de cobalt [i)] : le(s) composé(s) de nickel [i)] : le(s) composé(s) de cuivre [i)] : le(s) composé(s) de zinc [i)] : le(s) composé(s) de molybdène [i)] : de la glycine, [ii)] : du glycérol, [iii)] : de l'acide L-(+)ascorbique [iv)] : de l'acide éthylènediaminetétraacétique v)] : du tartrat de potassium-sodium (vi)] : de l'acide succinique (vii)] : de l'acide L-(+)-tartrique (viii)] dans un rapport pondéral de 0,01 à 1 : 0,01 à 1 : 0,2 à 3 : 0,01 à 0,6 : 0,02 à 2 : 0,15 à 6 : 0,02 à 1 : 0,01 à 2 : 0,01 à 1 : 0,1 à 3 : 0,001 à 0,8 : 0,1 à 2 : 0,2 à 8 : 0,01 à 2 : 0,01 à 3 : 0,01 à 10 : 0,001 à 2 : 0,1 à 2.

3. Le procédé selon la Revendication 1 ou 2, caractérisé en ce que la composition contient à titre de composé boré de l'acide borique [i)].

4. Le procédé selon les Revendications 1 à 3, caractérisé en ce que la composition contient à titre de composé fluoré [i)] du fluorure de sodium et/ou du trifluorure de vanadium.

5. Le procédé selon les Revendications 1 à 4, caractérisé en ce que la composition contient à titre de composé(s) de magnésium [i)] du sulfate de magnésium et/ou du chlorure de magnésium et/ou un (des) hydrate(s) de ceux-ci.

6. Le procédé selon les Revendications 1 à 5, caractérisé en ce que la composition contient à titre de composé de vanadium [i)] du vanadate d'ammonium et/ou du trifluorure de vanadium.

7. Le procédé selon les Revendications 1 à 6, caractérisé en ce que la composition contient à titre de composé(s) de manganèse [i)] du sulfate de manganèse et/ou du chlorure de manganèse et/ou un (des) hydrate(s) de ceux-ci.

8. Le procédé selon les Revendications 1 à 7, caractérisé en ce que la composition contient à titre de composé(s) de fer [i)] du sulfate de fer (II) et/ou du sulfate de fer(III) et/ou un (des) hydrate(s) de ceux-ci.

9. Le procédé selon les Revendications 1 à 8, caractérisé en ce que la composition contient à titre de composé(s) de cobalt [i)] du chlorure de cobalt et/ou du sulfate de cobalt et/ou un (des) hydrate(s) de ceux-ci.

10. Le procédé selon les Revendications 1 à 9, caractérisé en ce que la composition contient à titre de composé(s) de nickel [i)] du chlorure de nickel et/ou du sulfate de nickel et/ou un (des) hydrate(s) de ceux-ci.

11. Le procédé selon les Revendications 1 à 10, caractérisé en ce que la composition contient à titre de composé(s) de cuivre [i)] du sulfate de cuivre (II) ou un (des) hydrate(s) de ceux-ci.

12. Le procédé selon les Revendications 1 à 11, caractérisé en ce que la composition contient à titre de composé(s) de zinc [i)] du sulfate de Zinc et/ou un (des) hydrate(s) de ceux-ci.

13. Le procédé selon les Revendications 1 à 12, caractérisé en ce que la composition contient à titre de composé(s) de molybdène [i)] du molybdate d'ammonium et/ou du molybdate de sodium.

14. Le procédé selon les Revendications 1 à 13, caractérisé en ce que la composition contient à titre de sel d'acide éthylènediaminetétraacétique [v)] du sel disodique d'acide éthylènediaminetétraacétique et/ou un (des) hydrate(s) de ceux-ci.

15. Le procédé selon les Revendications 1 à 14, caractérisé en ce que la composition contient à titre de substance(s) d'ajustement du pH de la composition sur 1,9 à 4,0, un ou plusieurs acide(s) acceptable(s) du point de vue pharmaceutique dans la quantité nécessaire.

16. Le procédé selon les Revendications 1 à 15, caractérisé en ce que la composition est disponible sous une forme permettant l'administration orale, rectale ou vaginale.

17. Le procédé selon les Revendications 1 à 16, caractérisé en ce que la composition contient le(s) composé(s) boré(s) [i)] en une quantité de 0,01 à 1,0 % poids/volume, calculée sur le poids total de la composition.

18. Le procédé selon les Revendications 1 à 17, caractérisé en ce que la composition contient le(s) composé(s) fluoré(s) [i)] en une quantite de 0,01 à 1,0 % poids/volume, calculée sur le poids total de la composition.

19. Le procédé selon les Revendications 1 à 18, caractérisé en ce que la composition contient le(s) composé(s) de magnésium [i)] en une quantité de 0,2 à 3,0 % poids/volume, calculée sur le poids total de la composition.

20. Le procédé selon les Revendications 1 à 19, caractérisé en ce que la composition contient le(s) composé(s) de vanadium [i)] en une quantité de 0,001 à 0,6 % poids/volume, calculée sur le poids total de la composition.

21. Le procédé selon les Revendications 1 à 20, caractérisé en ce que la composition contient le(s) composé(s) de manganèse [i)] en une quantité de 0,02 à 2,0 % poids/volume, calculée sur le poids total de la composition.

22. Le procédé selon les Revendications 1 à 21, caractérisé en ce que la composition contient le(s) composé(s) de fer [i)] en une quantité de 0,15 à 6,0 % poids/volume, calculée sur le poids toal de la composition.

23. Le procédé selon les Revendications 1 à 22, caractérisé en ce que la composition contient le(s) composé(s) de cobalt [i)] en une quantité de 0,002 à 1,0 % poids/volume, calculée sur le poids toal de la composition.

24. Le procédé selon les Revendications 1 à 23, caractérisé en ce que la composition contient le(s) composé(s) de nickel [i)] en une quantité de 0,01 à 2,0 % poids/volume, calculée sur le poids toal de la composition.

25. Le procédé selon les Revendications 1 à 24, caractérisé en ce que la composition contient le(s) composé(s) de cuivre [i)] en une quantité de 0,01 à 1,0 % poids/volume, calculée sur le poids toal de la composition.

26. Le procédé selon les Revendications 1 à 25, caractérisé en ce que la composition contient le(s) composé(s) de zinc [i)] en une quantité de 0,10 à 3,0 % poids/volume, calculée sur le poids total de la composition.

27. Le procédé selon les Revendications 1 à 26, caractérisé en ce que la composition contient le(s) composé(s) de molybdène (i)] en une quantité de 0,001 à 0,8 % poids/volume, calculée sur le poids total de la composition.

28. Un procédé pour préparer un produit contenant une composition selon les Revendications 1 à 27 ainsi que de l'acide L-(+)-ascorbique en tant que préparation combinée destinée à un usage simultané, séparé ou successif dans la thérapie de la mucoviscidose et des syndrômes de douleur chronique dérivant de maladies locomotrices ou accompagnant des maladies d'origine tumorale, caractérisé en ce que l'on fait réagir les substances i) à viii) en milieu aqueux, et que l'on mélange éventuellement la solution ainsi obtenue avec une quantité supplémentaire d'acide L-(+)-ascorbique et/ou avec une ou plusieurs substance(s) et/ou composition(s) analgésique(s) et/ou narcotique(s) et/ou avec un ou plusieurs porteur(s), diluant(s) et/ou excipient(s) acceptable(s) du point de vue pharmaceutique, et que l'on forme une composition pharmaceutique.

29. Un procédé pour préparer un produit contenant une composition selon les Revendications 1 à 27 ou bien un produit selon la Revendication 28 et une ou plusieurs substance(s) et/ou composition(s) analgésiques et/ou narcotiques en tant que préparation combinée destinée à un usage simultané, séparé ou successif dans la thérapie de la mucoviscidose et des syndrômes de douleur chronique dérivant de maladies locomotrices ou accompagnant des maladies d'origine tumorale, caractérisé en ce que l'on fait réagir les substances i) a viii) en milieu aqueux, et que l'on mélange éventuellement la solution ainsi obtenue avec une quantité supplémentaire d'acide L-(+)-ascorbique et/ou avec une ou plusieurs substance(s) et/ou composition(s) analgésique(s) et/ou narcotique(s) et/ou avec un ou plusieurs porteur(s), diluant(s) et/ou excipient(s) acceptable(s) du point de vue pharmaceutique, et que l'on forme une composition pharmaceutique.
